# EUROPEAN PATENT APPLICATION

(11) **EP 4 223 742 A1**
(43) Date of publication of application: **09.08.2023**
(21) Application number: 21872851.7
(22) Date of filing: 17.09.2021
(51) Int. Cl.: C07D 209/46, A61K 31/4035, A61P 25/00, A61P 25/28, A61P 9/10, A23L 33/10, A23K 20/132

(54) **4-METHOXY-2-PHENETHYL ISOINDOLINE-1-ONE DERIVATIVE, AND COMPOSITION FOR TREATING NEUROLOGICAL DISORDERS, COMPRISING SAME**

(30) Priority: 23.09.2020 KR 20200123284
(71) Applicant: CNG Bio Co., Ltd., Cheongju-si, Chungcheongbuk-do 28420 (KR); Chungbuk National University Industry-Academic Cooperation Foundation, Cheongju-si, Chungcheongbuk-do 28644 (KR); Gachon University of Industry-Academic Cooperation Foundation, Seongnam-si, Gyeonggi-do 13120 (KR)
(72) Inventor: JUNG, Jaekyung, Cheongju-si Chungcheongbuk-do 28379 (KR); LEE, Mi Kyeong, Cheongju-si Chungcheongbuk-do 28162 (KR); LEE, Dae Hee, Cheongju-si Chungcheongbuk-do 28668 (KR); LEE, Jae Kang, Cheongju-si Chungcheongbuk-do 28610 (KR); KIM, Sun Yeou, Seoul 06280 (KR); YOON, Da Hye, Bucheon-si Gyeonggi-do 14787 (KR); HONG, Seong Min, Chungju-si Chungcheongbuk-do 27356 (KR)
(74) Representative: Snaith, James Michael
(86) International application number: PCT/KR2021/012793
(87) International publication number: WO 2022/065823

(57) **Abstract**

The present invention relates to: a novel 4-methoxy-2-phenethyl isoindolin-1-one derivative compound having the ability to promote the increase of nerve growth factor, the ability to promote the growth of nerve cells, and antineuritic activity; and a composition for preventing, improving and treating neurological diseases, comprising the same.

## Description

### Technical Field

The present invention relates to: a novel 4-methoxy-2-phenethyl isoindolin-1-one derivative compound having the ability to promote the increase of nerve growth factor, the ability to promote the growth of nerve cells, and antineuritic activity; and a composition for preventing, improving and treating neurological diseases, comprising the same.

### Background Art

Recently, with the rapid increase of the elderly population, the number of patients with various neurodegenerative brain diseases is increasing, and interest in treatment and prevention thereof is increasing. Neurodegenerative disease is a disease that causes several pathologies such as motor disorders, memory disorders, and cognitive disorders due to reduced or lost functions of nerve cells. A large number of nerve cells die every day not only in patients with neurological diseases but also in the brains of normal adults, and the number of nerve cells that die increases exponentially with aging.

Major diseases belonging to neurodegenerative diseases include Alzheimer's disease, Parkinson's disease, Lou Gehrig's disease, Huntington's disease, and the like, and the pathogenesis of these diseases has not been completely elucidated to date. Acetylcholinesterase inhibitors or NMDA (N-methyl-D-aspartate) receptor antagonists, etc., are used as a therapeutic agent for Alzheimer's disease, and L-dopa, dopamine agonists, MAO-B inhibitors, or COMT inhibitors, etc., are used as a therapeutic agent for Parkinson's disease, and dopamine D2 receptors and the like are used as a therapeutic agent for Huntington's disease. However, since all of the therapeutic agents target the neurotransmission process, the method using the therapeutic agents merely relieves symptoms rather than fundamental treatment. Therefore, new drugs capable of fundamental treatment have been continuously required.

On the other hand, it is known that neurological diseases are closely related to the deterioration of the function of brain nerve cells and the death of nerve cells (Korean Patent No. 10-0935615). The present inventors have found that the ability to promote the increase of nerve growth factor, the ability to promote the growth of nerve cells, and antineuritic activity of a 4-methoxy-2-phenethyl isoindolin-1-one derivative were excellent, thereby completing the present invention.

### Prior Art Document

### Patent Document

(Patent Document 1) Korean Patent No. 10-0935615

### Non-Patent Document

(Non-Patent Document 1) Yuan and Yankner, Nature. 407, 802-809, 2000

### Detailed Description of Invention

### Technical Problem

An object of the present invention is to provide a novel compound and a pharmaceutically acceptable salt thereof having the ability to promote the increase of nerve growth factor, the ability to promote the growth of nerve cells, and antineuritic activity.

In addition, another object of the present invention is to provide a composition for preventing, improving or treating neurological diseases, comprising the compound and pharmaceutically acceptable salt thereof.

### Solution to Problem

The present invention provides a compound represented by Formula 9 below or a pharmaceutically acceptable salt thereof. 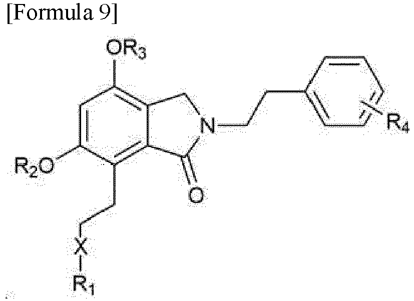 in the formula, R₁ is hydrogen, alkyl, alkenyl, alkynyl, hydroxy, alkoxy, hydroxyalkyl, alkoxyalkyl, or -N(Ra)(Rb); R₂, R₃, R₄, Ra and Rb are each independently hydrogen or alkyl; and X is a single bond or a double bond.

In addition, the present invention provides a composition for preventing, improving or treating neurological diseases, comprising the compound represented by Formula 9 and pharmaceutically acceptable salt thereof.

### Effects of Invention

The novel compound of the present invention has the ability to promote the increase of nerve growth factor, the ability to promote the growth of nerve cells, and antineuritic activity.

Therefore, a composition comprising the novel compound of the present invention can be used as a pharmaceutical composition, a food composition and a feed composition having effects of preventing, improving and treating neurological diseases.

### Brief Description of Drawings

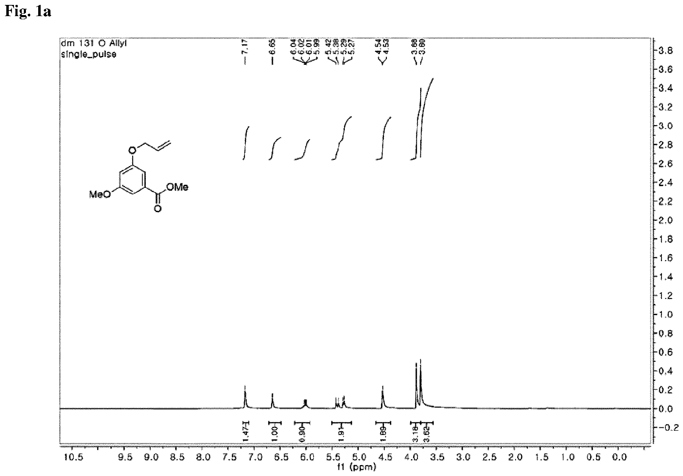

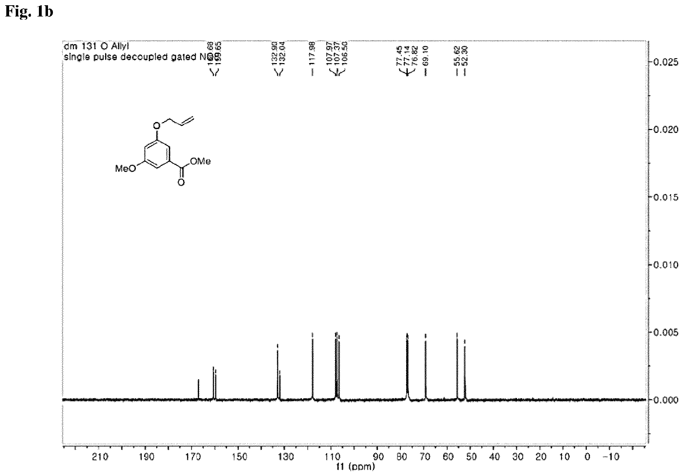

Figs. 1a and 1b show the results of ¹H NMR and ¹³C NMR analysis of the compound of Formula 1.

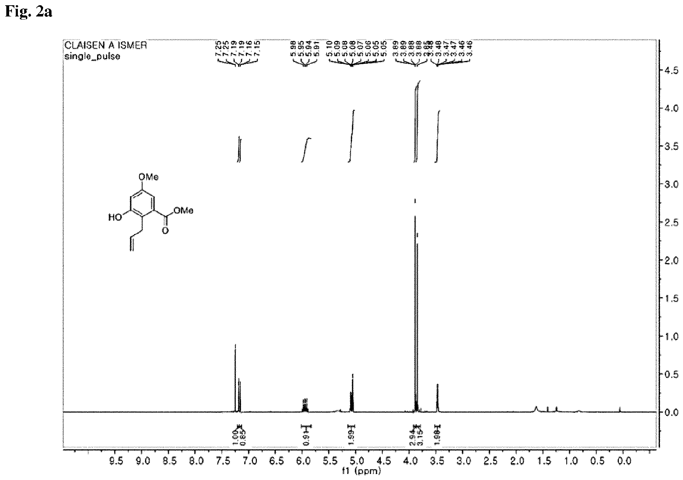

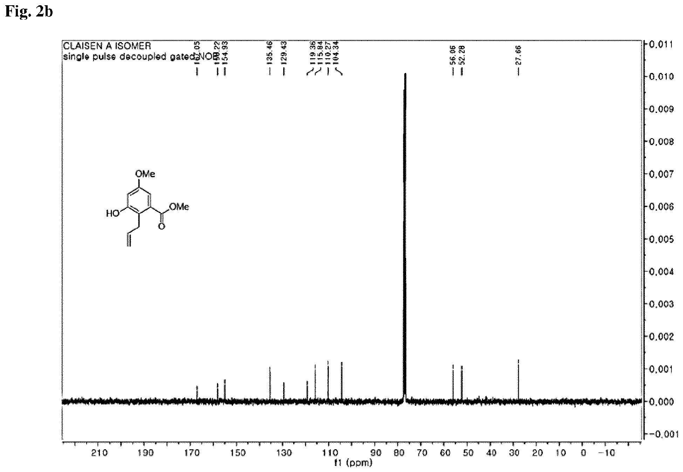

Figs. 2a and 2b show the results of ¹H NMR and ¹³C NMR analysis of the compound of Formula 2.

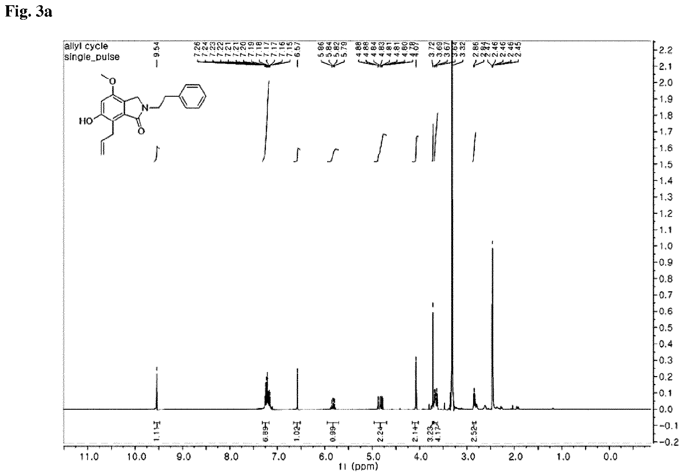

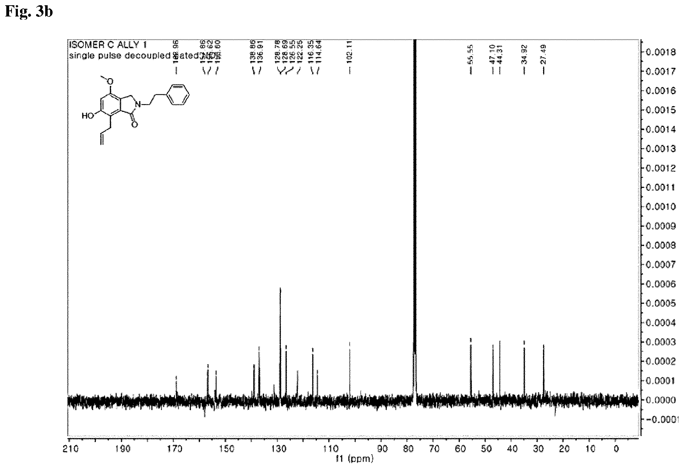

Figs. 3a and 3b show the results of ¹H NMR and ¹³C NMR analysis of the compound of Formula 3.

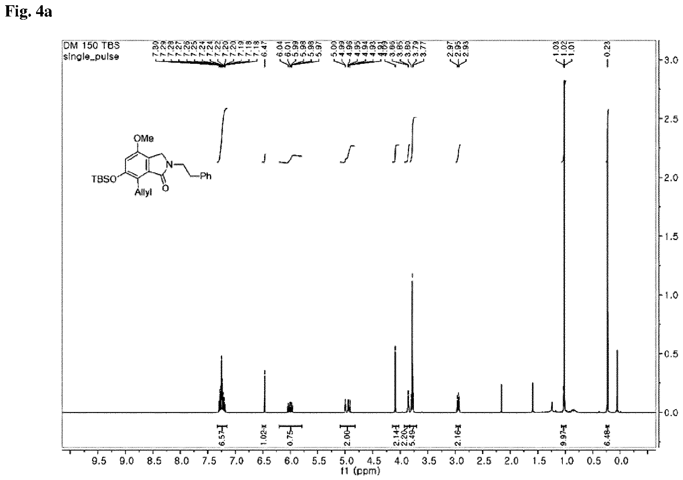

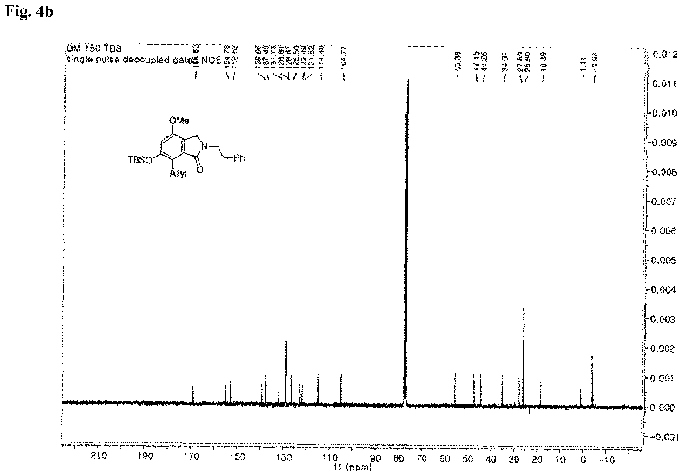

Figs. 4a and 4b show the results of ¹H NMR and ¹³C NMR analysis of the compound of Formula 4.

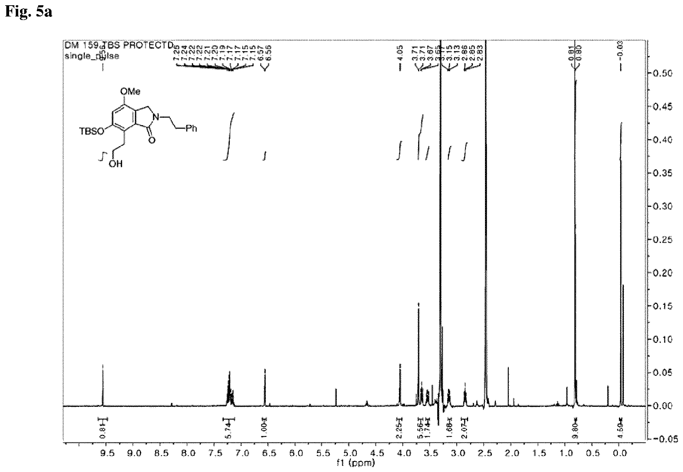

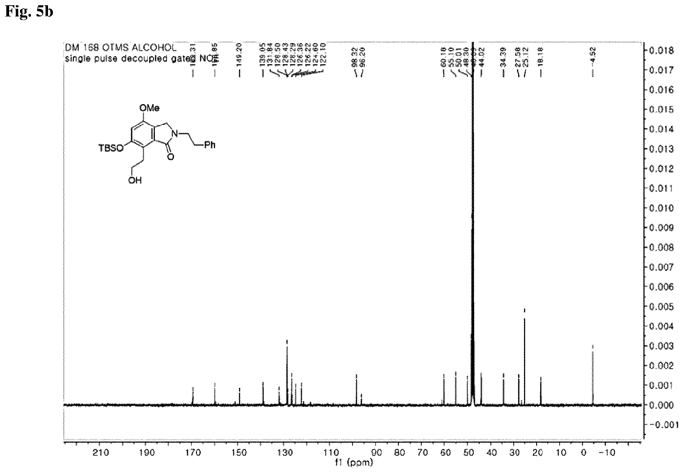

Figs. 5a and 5b show the results of ¹H NMR and ¹³C NMR analysis of the compound of Formula 5.

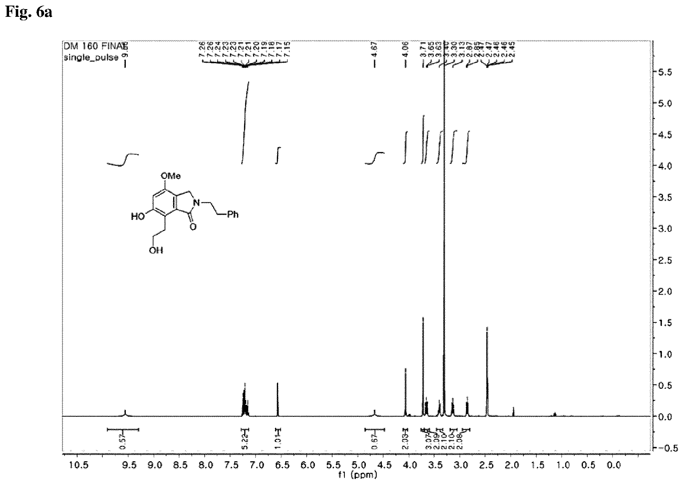

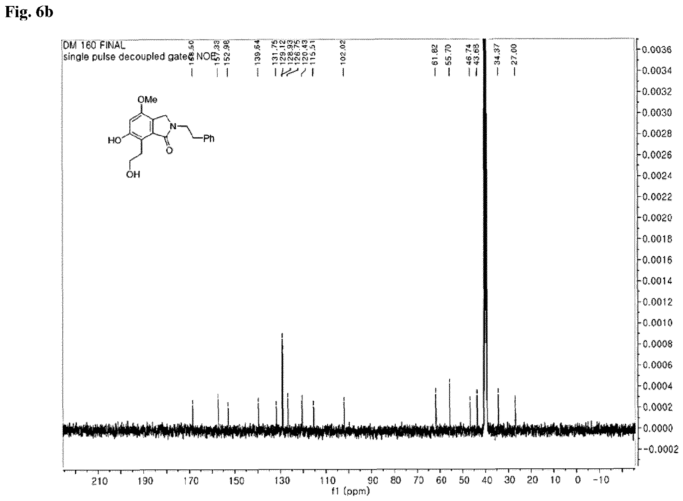

Figs. 6a and 6b show the results of ¹H NMR and ¹³C NMR analysis of the compound of Formula 6.

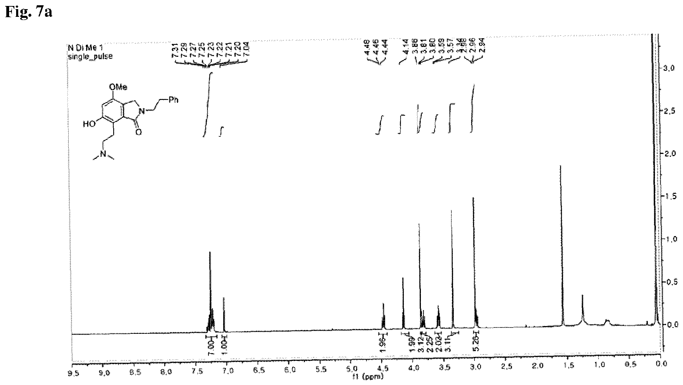

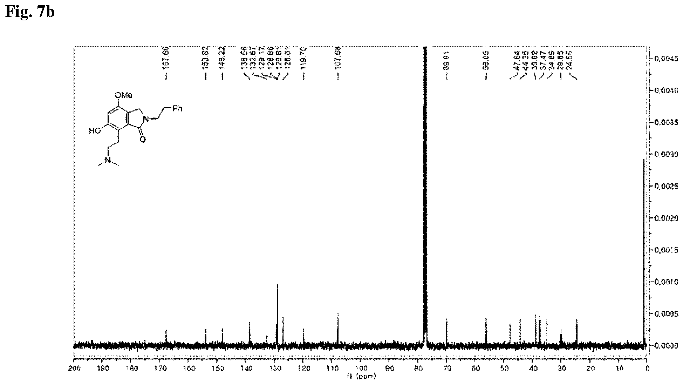

Figs. 7a and 7b show the results of ¹H NMR and ¹³C NMR analysis of the compound of Formula 7.

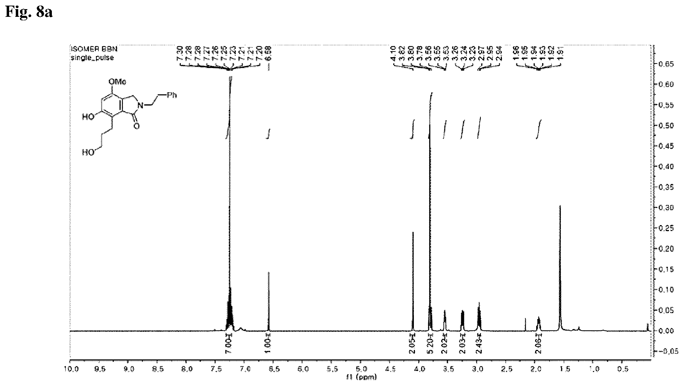

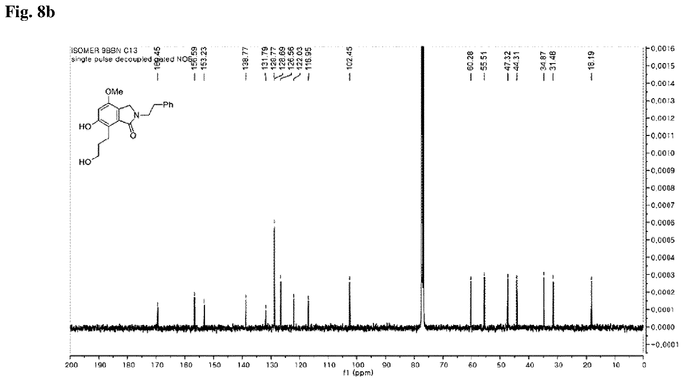

Figs. 8a and 8b show the results of ¹H NMR and ¹³C NMR analysis of the compound of Formula 8.
Fig. 9 is a graph showing the amount of nerve growth factor produced in C6 glioma cells.
Fig. 10 is a graph showing the results of cytotoxicity evaluation in C6 glioma cells.
Figs. 11a to 11c show the results obtained by measuring neurite growth in N2a cells.
Fig. 12 is a graph showing the results of cytotoxicity evaluation in N2a cells.
Fig. 13 is a graph showing the amount of NO production measured in BV2 cells.
Fig. 14 is a graph showing the results of cytotoxicity evaluation in BV2 cells.

### Best Mode for Carrying out the Invention

Hereinafter, with reference to the accompanying drawings, embodiments and examples of the present disclosure will be described in detail so that those of ordinary skill in the art to which the present invention belongs can easily practice the present invention. However, the present disclosure may be implemented in various forms and is not limited to the embodiments and examples described herein.

Throughout the present specification, when a certain part "includes" a certain component, it means that other components may be further included, rather than excluding other components, unless otherwise stated.

The present invention provides a compound represented by Formula 9 below or a pharmaceutically acceptable salt thereof. 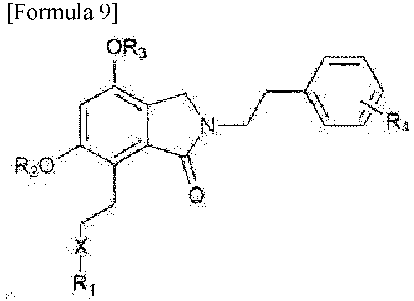 in the formula, R₁ is hydrogen, alkyl, alkenyl, alkynyl, hydroxy, alkoxy, hydroxyalkyl, alkoxyalkyl, or -N(Ra)(Rb); R₂, R₃, R₄, Ra and Rb are each independently hydrogen or alkyl; and X is a single bond or a double bond.

In one embodiment, R₁ may be hydrogen, alkyl, alkenyl, alkynyl, hydroxy, alkoxy, hydroxyalkyl, alkoxyalkyl, or -N(Ra)(Rb); Ra and Rb may be each independently hydrogen or alkyl; R₂ and R₄ may be hydrogen; R₃ may be alkyl; and X may be a single bond or a double bond.

In one embodiment, R₁ may be C₁-C₆ alkyl, hydroxy, C₁-C₆ hydroxyalkyl, or - N(Ra)(Rb); Ra and Rb may be each independently C₁-C₆ alkyl; R₂ and R₄ may be hydrogen; R₃ may be C₁-C₆ alkyl; and X may be a single bond or a double bond.

In one embodiment, R₁ may be hydroxy, -CH₂OH, or -N(CH₃)(CH₃); R₂ and R₄ may be hydrogen; R₃ may be methyl; and X may be a single bond.

In one embodiment, R₁ may be methyl; R₂ and R₄ may be hydrogen; R₃ may be methyl; and X may be a double bond.

As used herein, the term "pharmaceutically acceptable salt" refers to a salt according to one aspect of the present invention that is pharmaceutically acceptable and has the desired pharmacological activity of the parent compound. In addition, pharmaceutically acceptable salts herein are intended to refer to all salts that can be used not only in pharmaceutical compositions, but also in cosmetic compositions or food compositions. The salt is not particularly limited as long as it is pharmaceutically acceptable, and for example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, and the like can be used.

The present invention provides a compound selected from the group consisting of compounds of the following formulas or a pharmaceutically acceptable salt thereof. 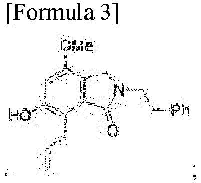 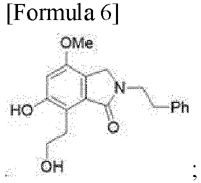 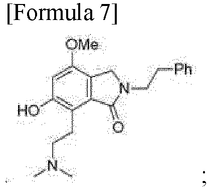 and

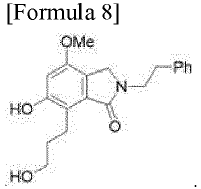

In one embodiment, the compounds of Formulas 3 and 6 above may be prepared according to Reaction Scheme 1 below.

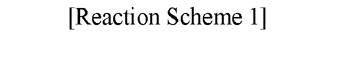

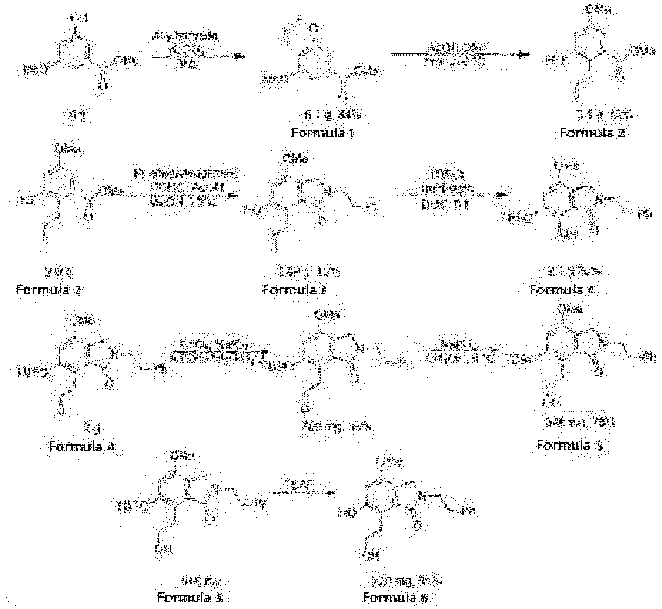

In one embodiment, the compound of Formula 7 above may be prepared according to Reaction Scheme 2 below.

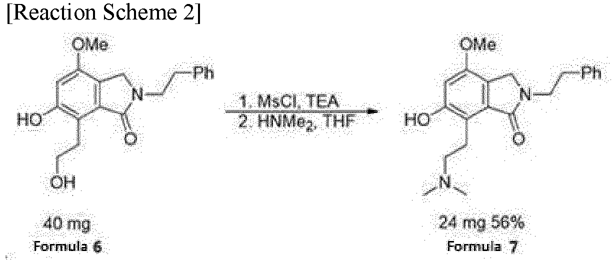

In one embodiment, the compound of Formula 8 above may be prepared according to Reaction Scheme 3 below.

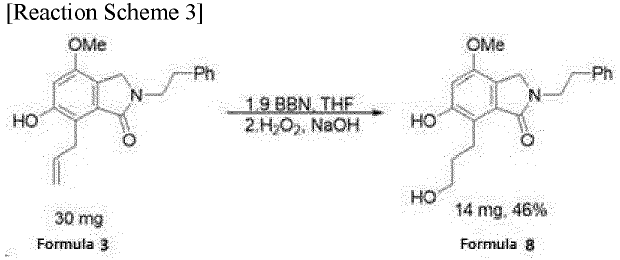

The present invention provides a pharmaceutical composition for preventing or treating neurological diseases, comprising the compound represented by Formula 9 or pharmaceutically acceptable salt thereof. In addition, the present invention provides a method for preventing or treating neurological diseases, comprising administering the pharmaceutical composition to a subject. The subject may be a mammal, including a human, who has been diagnosed with a neurological disease or is likely to develop a neurological disease.

In one embodiment, the compound represented by Formula 9 may promote the increase of nerve growth factor or promote the growth of nerve cells. In another embodiment, the compound represented by Formula 9 may have antineuritic activity.

The pharmaceutical composition of the present invention may comprise a pharmaceutically acceptable additive. The additive includes a stabilizer, a surfactant, a lubricant, a solubilizer, a buffering agent, a sweetener, a base, an adsorbent, a corrigent, a binder, a suspending agent, a curing agent, an antioxidant, a brightener, a fragrance, a flavoring agent, a pigment, a coating agent, a wetting agent, a moisture adjusting agent, a filler, an antifoaming agent, a cooling agent, a chewing agent, an antistatic agent, a coloring agent, a dragee, a tonicity agent, a softener, an emulsifier, an adhesive, a thickener, a foaming agent, a pH adjusting agent, an excipient, a dispersing agent, a disintegrant, a waterproofing agent, an antiseptic, a preservative, a solubilizing agent, a solvent, a flowing agent, and the like, but is not limited thereto.

The pharmaceutical composition of the present invention can be parenterally administered or orally administered depending on the desired method, and the dosage varies depending on the patient's weight, age, sex, health condition, diet, administration time, administration method, excretion rate, and severity of the disease. In addition, the therapeutically effective amount of the composition may vary depending on the administration method, the target site, and the condition of the patient, and when used in the human body, the dosage should be determined in an appropriate amount in consideration of safety and efficiency.

The present invention provides a food composition for preventing or improving neurological diseases, comprising the compound represented by Formula 9 or pharmaceutically acceptable salt thereof.

In one embodiment, the food composition may further comprise various nutrients, vitamins, minerals (electrolytes), flavors such as synthetic flavors and natural flavors, coloring agents and enhancers (cheese, chocolate, etc.), pectic acid and a salt thereof, alginic acid and a salt thereof, organic acids, protective colloidal thickeners, pH adjusting agents, stabilizers, antiseptics, glycerin, alcohol, carbonation agents used in carbonated beverages, and the like.

In one embodiment, the food composition includes a form such as a pill, a powder, a granule, a precipitate, a tablet, a capsule, a liquid, a paste, a gel, or a jelly, and the food to which the composition can be added includes, for example, various types of foods, such as beverages, chewing gum, tea, vitamin complexes, health supplementary food, and the like.

The formulation of the food composition is not particularly limited, but may be formulated into, for example, a tablet, a granule, a powder, a liquid such as a drink, a caramel, a gel, a bar, and the like. The food composition of each formulation may be formulated without difficulty by those of ordinary skill in the art depending on the formulation or purpose of use by selecting and blending ingredients commonly used in the field in addition to the active ingredient.

The present invention provides a feed composition for preventing or improving neurological diseases, comprising the compound represented by Formula 9 or pharmaceutically acceptable salt thereof.

The feed composition may be ingested by all non-human animals, such as non-human primates, sheep, dogs, cattle, horses, and the like.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition, and "improvement" refers to any action that at least reduces a parameter related to a condition, for example, the severity of a symptom, by administration of a composition.

In one embodiment, the neurological disease may be a neurological disease associated with or accompanied by reduced nerve growth factor activity, decreased nerve growth factor, nerve cell death, neuritis or reduced function of nerve cells. Preferably, the neurological disease may be Alzheimer's disease, dementia, Parkinson's disease, epilepsy, neurological disorder, peripheral neuropathy, stroke or ischemic brain disease.

### Definition

Unless defined otherwise, all technical terms used herein have the same meaning as commonly understood by those of ordinary skill in the art to which the present invention belongs. Moreover, numerical values described herein are considered to include the meaning of "about" unless explicitly stated otherwise. Definitions of moieties and substituents as used herein are provided below. Unless specified otherwise, each moiety has the following definitions and is used to have the same meaning as commonly understood by those of ordinary skill in the art.

As used herein, the term "Cx-y" or "Cx-Cy," when used in conjunction with a chemical moiety such as acyl, acyloxy, alkyl, haloalkyl, cycloalkyl, alkenyl, alkynyl or alkoxy, is intended to include a group containing from x to y carbons in the chain. C₀ alkyl represents hydrogen when the substituent is in the terminal position or a bond when the substituent is in the internal position. In addition, for example, a C₁-C₆ alkyl group contains from 1 to 6 carbon atoms in the chain.

As used herein, the term "alkyl" is a hydrocarbon having unsubstituted or substituted primary, secondary, tertiary and/or quaternary carbon atoms, and includes a saturated aliphatic group which may be straight-chain, branched or cyclic, or a combination thereof. For example, an alkyl group may have from 1 to 20 carbon atoms (i.e., C₁-C₂₀ alkyl), from 1 to 10 carbon atoms (i.e., C₁-C₁₀ alkyl), or from 1 to 6 carbon atoms (i.e., C₁-C₆ alkyl). Unless defined otherwise, in preferred embodiments, an alkyl refers to C₁-C₆ alkyl. Examples of suitable alkyl groups may include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, - CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), and the like, but are not limited thereto.

Moreover, as used throughout the specification, examples and claims, the term "alkyl" is intended to include both unsubstituted and substituted alkyl groups, the latter of which refers to an alkyl moiety having a substituent that replaces a hydrogen on at least one carbon of the hydrocarbon backbone, which includes a haloalkyl group such as trifluoromethyl and 2,2,2-trifluoroethyl, and the like.

As used herein, the term "alkenyl" is a hydrocarbon that has primary, secondary, tertiary and/or quaternary carbon atoms, includes straight-chain, branched and cyclic groups, or a combination thereof, and has at least one unsaturated region, i.e., a carbon-carbon sp² double bond. For example, an alkenyl group may have from 2 to 20 carbon atoms (i.e., C₂-C₂₀ alkenyl), from 2 to 12 carbon atoms (i.e., C₂-C₁₂ alkenyl), from 2 to 10 carbon atoms (i.e., C₂-C₁₀ alkenyl), or from 2 to 6 carbon atoms (i.e., C₂-C₆ alkenyl). Examples of suitable alkenyl groups may include vinyl (-CH=CH₂), allyl (-CH₂CH=CH₂), cyclopentenyl (-C₅H₇), and 5-hexenyl (-CH₂CH₂CH₂CH₂CH=CH₂), but are not limited thereto.

As used herein, the term "alkynyl" is a hydrocarbon that has primary, secondary, tertiary and/or quaternary carbon atoms, includes straight-chain, branched and cyclic groups, or a combination thereof, and has at least one carbon-carbon sp triple bond. For example, an alkynyl group may have from 2 to 20 carbon atoms (i.e., C₂-C₂₀ alkynyl), from 2 to 12 carbon atoms (i.e., C₂-C₁₂ alkynyl), from 2 to 10 carbon atoms (i.e., C₂-C₁₀ alkynyl), or from 2 to 6 carbon atoms (i.e., C₂-C₆ alkynyl). Examples of suitable alkynyl groups may include acetylenic (-C=CH) and propargyl (-CH₂C≡CH), but are not limited thereto.

As used herein, the term "alkoxy" refers to a group in which an alkyl group is attached to the parent compound through an oxygen atom, which may be represented by -O-alkyl, wherein the alkyl group is as defined herein and may be unsubstituted or substituted. The alkyl group of an alkoxy group may have, for example, from 1 to 20 carbon atoms (i.e., C₁-C₂₀ alkoxy), from 1 to 12 carbon atoms (i.e., C₁-C₁₂ alkoxy), from 1 to 10 carbon atoms (i.e., C₁-C₁₀ alkoxy), or from 1 to 6 carbon atoms (i.e., C₁-C₆ alkoxy). Examples of suitable alkoxy groups may include methoxy (-O-CH₃ or -OMe), ethoxy (-OCH₂CH₃ or -OEt), and t-butoxy (-OC(CH₃)₃ or -O-tBu), and the like, but are not limited thereto.

As used herein, the term "hydroxyalkyl" refers to an alkyl group substituted with a hydroxyl group, and may be represented by -alkyl-OH.

As used herein, the term "alkoxyalkyl" refers to an alkyl group substituted with an alkoxy group, as defined herein, and may be represented by -alkyl-O-alkyl.

Hereinafter, the present invention will be described in more detail through the examples, but the following examples are for illustrative purposes only and are not intended to limit the scope of the present invention.

### [Example 1] Preparation of compound of Formula 1

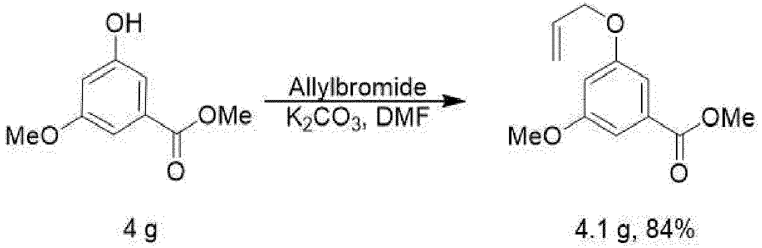

To a solution of methyl 3-hydroxy-5-methoxybenzoate (4 g, 21.96 mmol) in DMF was added 2.5 equivalents of potassium carbonate, and allyl bromide (6.64 g, 54.89 mmol) was added dropwise at room temperature. The reaction mixture was stirred for 15 hours at room temperature. The reaction was quenched by the addition of a saturated aqueous NaHCOs solution (30 mL) and washed with CHCl₃ (3 × 30 mL), and then the combined organic layers were dried over MgSO₄, filtered and concentrated. The crude product was purified by silica gel column chromatography (hexane/Et₂O, 20:1) to obtain methyl 3-(allyloxy)-5-methoxybenzoate (4.1 g, 84%) represented by Formula 1 as a white solid.

¹H NMR (400 MHz, CDCl₃); δ 7.17 (s, 1H), 6.65 (s, 1H), 6.01 (dd, *J* = 11.4, 5.4 Hz, 1H), 5.34 (dd, *J* = 49.4, 13.4 Hz, 2H), 4.53 (d, *J* = 5.1 Hz, 2H), 3.84 (d, *J* = 32.6 Hz, 3H), 3.80 (s, 3H). ¹³C NMR (100 MHz, CDCl₃); δ 168.85, 160.68, 159.65, 132.90, 132.04, 117.98, 107.97, 107.37, 106.50, 69.10, 55.62, 52.30.

### [Example 2] Preparation of compound of Formula 2

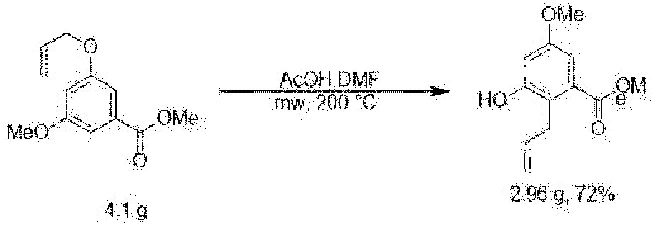

Methyl 3-(allyloxy)-5-methoxybenzoate (4.1 g, 18.45 mmol) represented by Formula 1 was refluxed in DMF (10 mL) and AcOH (2 ml) for 1 day. The cooled reaction mixture was concentrated in vacuo and dissolved in AcOEt (30 ml). The organic layer was washed with a saturated aqueous NaCl solution (100 ml), and then dried over MgSO_{4,} and concentrated. The crude product was purified by silica gel column chromatography using a 1:3 mixture of AcOEt and hexane as an eluent to obtain methyl 2-allyl-3-hydroxy-5-methoxybenzoate (2.96 g, 72%) represented by Formula 2 as a white solid.

¹H NMR (400 MHz, CDCl₃); δ 7.19 (d, *J* = 1.4 Hz, 1H), 7.16 (d, *J* = 1.4 Hz, 1H), 5.94 (dd, *J* = 16.8, 10.4 Hz, 1H), 5.14 - 5.02 (m, 2H), 3.92 - 3.86 (m, 3H), 3.86 (d, *J* = 6.5 Hz, 3H), 3.47 (dt, *J* = 6.1, 1.6 Hz, 2H). ¹³C NMR (100 MHz, CDCl₃); δ 167.05, 158.22, 154.93, 135.46, 129.43, 119.36, 115.84, 110.27, 104.34, 56.06, 52.28, 27.66.

### [Example 3] Preparation of compound of Formula 3

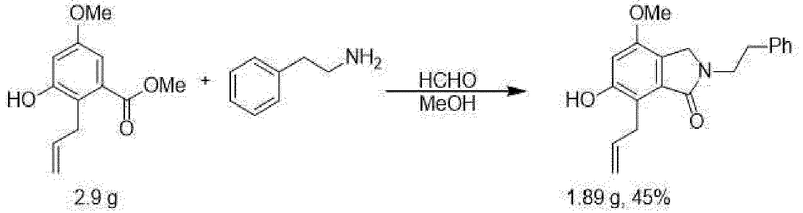

To a solution of methyl 2-allyl-3-hydroxy-5-methoxybenzoate (2.9 g, 13.05 mmol) represented by Formula 2 in methanol (10 ml) was added 2 equivalents (3.16 g, 26.12 mmol) of phenylethanamine, and a 30% formaldehyde solution (4.0 mL) was added dropwise at room temperature. The reaction mixture was warmed to 60 °C and stirred for 12 hours. After confirming the reaction completion by TLC, the reaction mixture was cooled back to room temperature and quenched by the slow addition of a saturated aqueous solution of sodium hydrogen carbonate. The aqueous layer was extracted three times with EtOAc, and the combined organic layers were washed with brine, dried over sodium sulfate, evaporated, and then purified by column chromatography to obtain 7-allyl-6-hydroxy-4-methoxy-2-phenethyl isoindolin-1-one (1.89 g, 45%) represented by Formula 3 as a white solid.

¹H NMR (400 MHz, DMSO-*d*₆); δ 9.54 (s, 1H), 7.32 - 7.17 (m, 5H), 6.57 (s, 1H), 5.83 (dd, *J =* 17.1, 10.1 Hz, 1H), 4.96 - 4.70 (m, 2H), 4.07 (s, 2H), 3.72 (s, 3H), 3.69 - 3.62 (m, 4H), 2.85 (t, *J =* 7.4 Hz, 2H). ¹³C NMR (100 MHz, CDCl₃); δ 168.96, 157.86, 156.62, 153.60, 138.86, 136.91, 128.78, 128.69, 126.55, 122.25, 116.35, 114.64, 102.11, 55.55, 47.10, 44.31, 34.92, 27.49.

### [Example 4] Preparation of compound of Formula 4

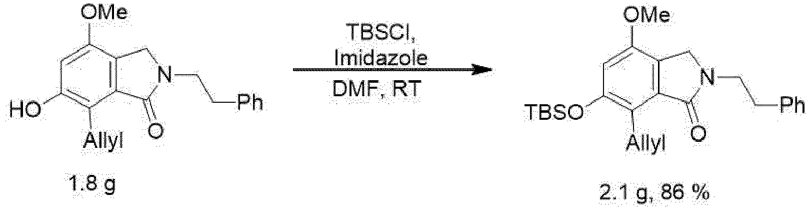

To a solution of 7-allyl-6-hydroxy-4-methoxy-2-phenethyl isoindolin-1-one (1.8 g, 5.57 mmol) represented by Formula 3 and 1H imidazole (2.5 equivalents) in CH₂Cl₂ (25 mL) was added tert-butyldimethylchlorosilane (1.67 g, 11.14 mmol). The reaction mixture was stirred for 2 hours at room temperature. The reaction was quenched by the addition of a saturated aqueous NaHCO₃ solution (30 mL) and washed with CH₂Cl₂ (3 × 30 mL). The combined organic layers were dried over MgSO₄, filtered and concentrated. The crude product was purified by silica gel column chromatography (hexane/Et₂O, 20:1) to obtain 7-allyl-6-((tert-butyldimethylsilyl)oxy)-4-methoxy-2-phenethyl isoindolin-1-one (2.1 g, 86%) represented by Formula 4 as a white solid.

¹H NMR (400 MHz, CDCl₃); δ 7.33 - 7.15 (m, 5H), 6.47 (s, 1H), 6.00 (ddt, *J* = 16.3, 10.1, 6.2 Hz, 1H), 5.09 - 4.83 (m, 2H), 4.09 (s, 2H), 3.86 (d, *J* = 6.1 Hz, 2H), 3.82 - 3.71 (m, 5H), 2.99 - 2.91 (m, 2H), 1.07 - 0.99 (m, 9H), 0.23 (s, 6H). ¹³C NMR (100 MHz, CDCl₃); δ 168.82, 154.78, 152.62, 138.96, 137.49, 131.73, 128.74, 126.50, 122.49, 121.52, 114.48, 104.77, 55.38, 47.15, 44.26, 34.91, 27.69, 25.90, 18.39, 1.11, -3.93.

### [Example 5] Preparation of compound of Formula 5

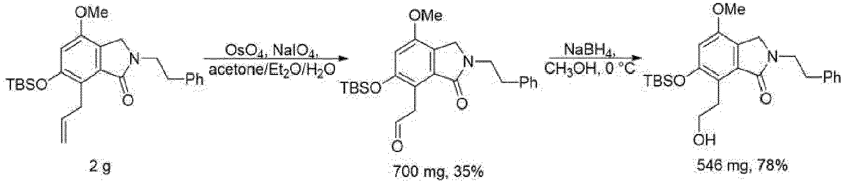

7-allyl-6-((tert-butyldimethylsilyl)oxy)-4-methoxy-2-phenethyl isoindolin-1-one (2 g, 4.55 mmol) represented by Formula 4 was added to Et₂O (24 mL), and then to the solution was added a 0.05M solution of OsO₄ (2.5 equivalents) in acetone. The resulting dark brown solution was stirred for 10 minutes, and water (24mL) was added, and then finely powdered NaIO₄ (4.85 g, 22.77 mmol) was added in 5 portions over 5 hours. The tan slurry was stirred for additional 3 hours, and then diluted with Et₂O, and the layers were separated. The organic layer was washed with saturated brine, dried over Na₂SO₄, filtered, and then concentrated in vacuo. The crude product was dissolved in CH₃OH (6.0 mL), cooled to 0 °C, and then treated with NaBH₄ (121 mg, 3.18 mmol). After 30 minutes, the excess hydride was quenched by the addition of 10% HCl, and the resulting mixture was extracted with Et₂O. The combined organic extracts were washed with saturated brine, dried over Na₂SO₄, filtered through a SiO₂ plug (1.0 in.) over a pad of Celite, and then concentrated in vacuo to obtain 6-((tertbutyldimethylsilyl)oxy)-7-(2-hydroxyethyl)-4-methoxy-2-phenethyl isoindolin-1-one (546 mg, 78%) represented by Formula 5 as a white solid.

¹H NMR (400 MHz, DMSO-*d*₆); δ 9.56 (s, 1H), 7.33 - 7.12 (m, 5H), 6.56 (d, *J* = 3.9 Hz, 1H), 4.05 (s, 2H), 3.72 - 3.62 (m, 5H), 3.57 - 3.51 (m, 2H), 3.14 (d, *J* = 7.1 Hz, 2H), 2.85 (t, *J* = 7.3 Hz, 2H), 0.80 (d, *J* = 3.3 Hz, 9H), -0.03 (s, 6H). ¹³C NMR (100 MHz, CD₃OD); δ 169.31, 159.85, 149.20, 139.05, 131.84, 124.60, 122.10, 98.32, 96.20, 60.18, 55.10, 50.01, 48.30, 48.09, 44.02, 34.39, 27.58, 25.12, 18.18, -4.52.

### [Example 6] Preparation of compound of Formula 6

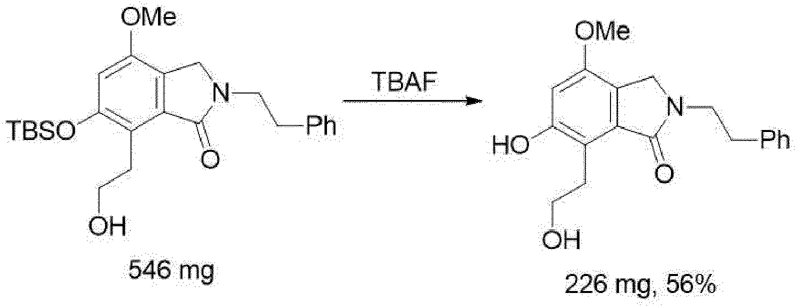

To a solution of 6-((tert-butyldimethylsilyl)oxy)-7-(2-hydroxyethyl)-4-methoxy-2-phenethyl isoindolin-1-one (546 mg, 1.23 mmol) represented by Formula 5 in THF at 0 °C was added dropwise 1.85 mL (1.85 mmol) of a 1.0 M solution of tetra-n-butylammonium fluoride in THF. The solution was warmed to room temperature for 1 hour and added to a saturated solution of NH₄Cl. The mixture was extracted with Et₂O, and then the combined organic layers were dried over Na₂SO₄, filtered, and then concentrated in vacuo to give an oil, which was purified by SiO₂ chromatography (50% EtOAc/hexane) to obtain 6-hydroxy-7-(2-hydroxyethyl)-4-methoxy-2-phenethyl isoindolin-1-one (226 mg, 56%) represented by Formula 6.

¹H NMR (400 MHz, DMSO-*d*₆); δ 9.56 (s, 1H), 7.29 - 7.13 (m, 5H), 6.57 (s, 1H), 4.67 (s, 1H), 4.06 (s, 2H), 3.71 (s, 3H), 3.65 (t, *J* = 7.4 Hz, 2H), 3.39 (d, *J* = 7.7 Hz, 2H), 3.13 (t, *J* = 7.5 Hz, 2H), 2.85 (t, *J* = 7.4 Hz, 2H). ¹³C NMR (100 MHz, DMSO-*d*₆); δ 168.50, 157.33, 152.98, 139.64, 131.75, 129.12, 128.93, 126.75, 120.43, 115.51, 102.02, 61.82, 55.70, 46.74, 43.68, 34.37, 27.00.

### [Example 7] Preparation of compound of Formula 7

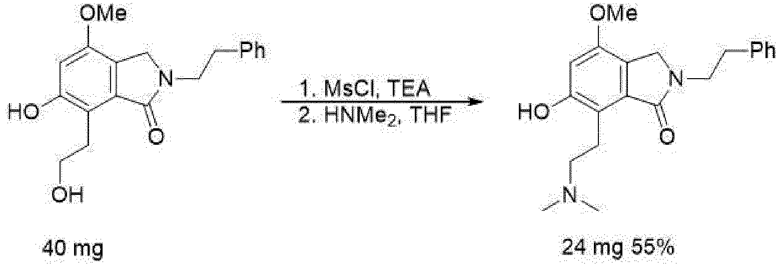

6-hydroxy-7-(2-hydroxyethyl)-4-methoxy-2-phenethyl isoindolin-1-one (40 mg, 0.12 mmol) represented by Formula 6 was added to THF at room temperature, and then MsCl followed by TEA were added dropwise to the reaction mixture, and then stirred for 3 hours. Upon formation of the starting material, the reaction mixture was cooled to 0 °C, and dimethylamine in THF was added to the reaction, and then the reaction mixture was stirred for 6 hours at 60 °C. The reaction was quenched with brine (30 mL) and washed with CHCl₃ (3 × 30 mL). The combined organic layers were dried over MgSO₄, filtered and concentrated. The crude product was purified by column chromatography to obtain 7-(2-(dimethylamino)ethyl)-6-hydroxy-4-methoxy-2-phenethyl isoindolin-1-one (24 mg, 55%) represented by Formula 7 as a white solid.

¹H NMR (400 MHz, CDCl₃); δ 7.33 - 7.16 (m, 5H), 7.04 (s, 1H), 4.46 (t, *J* = 7.1 Hz, 2H), 4.14 (s, 2H), 3.86 (s, 3H), 3.81 (t, *J* = 7.4 Hz, 2H), 3.57 (t, *J* = 7.1 Hz, 2H), 3.34 (s, 3H), 3.01 - 2.93 (m, 5H). ¹³C NMR (100 MHz, CDCl₃); δ 167.58, 153.75, 148.15, 138.49, 132.59, 129.09, 128.76, 126.74, 119.62, 107.60, 69.84, 55.98, 47.56, 44.28, 38.75, 37.40, 34.81, 29.78, 24.48.

### [Example 8] Preparation of compound of Formula 8

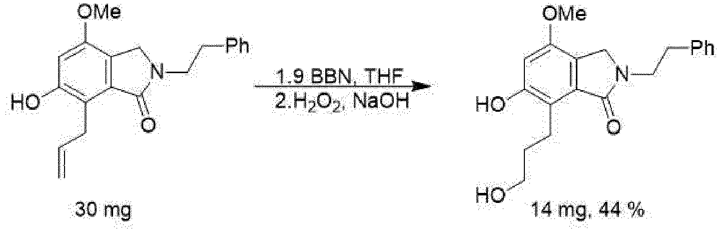

To a solution of 7-allyl-6-hydroxy-4-methoxy-2-phenethyl isoindolin-1-one (30 mg, 0.09 mmol) represented by Formula 3 in THF at 0 °C was added dropwise 9 BBN (2.5 equivalents) in THF. The reaction mixture was stirred at room temperature for 5 hours. The reaction was quenched by the addition of a saturated aqueous solution of H₂O₂ (5 equivalents) and NaOH (3 mL, 2 M) and stirred for 3 hours. The reaction mixture was washed with CHCl₃ (3 × 30 mL). The combined organic layers were dried over MgSO₄, filtered and concentrated. The crude product was purified by silica gel column chromatography (hexane/Et₂O, 5:1) to obtain 6-hydroxy-7-(3-hydroxypropyl)-4-methoxy-2-phenethyl isoindofin-1-one (14 mg, 44 %) represented by Formula 8 as a white solid.

¹H NMR (400 MHz, CDCl₃) δ 7.34 - 7.09 (m, 1H), 6.58 (s, 1H), 4.10 (s, 1H), 3.89 - 3.70 (m, 1H), 3.55 (t, *J* = 5.4 Hz, 1H), 3.35 - 3.21 (m, 1H), 3.10 - 2.93 (m, 1H), 1.93 (td, *J* = 11.1, 6.1 Hz, 1H). ¹³C NMR (100 MHz, CDCl₃) δ 169.45, 156.59, 153.23, 138.77, 131.79, 128.73, 126.56, 122.03, 116.95.

### [Experimental Example 1] Evaluation of ability to promote increase of nerve growth factor and cytotoxicity

In order to measure the amount of nerve growth factor (NGF) produced in the cell medium and to confirm the effect on cell viability when cells were treated with the compounds of the present invention, the following experiments were performed.

### Evaluation of ability to promote increase of nerve growth factor (NGF)

Rat astrocyte-derived C6 glioma cells were dispensed in DMEM culture solution at a concentration of 1 × 10⁵ cells/well in a 24 well plate and stabilized overnight, and then treated with the compounds of Formulas 3, 6, 7, and 8 at a concentration of 10 µM, and reacted in a 37 °C incubator for 24 hours. The treated medium was collected and centrifuged, and then absorbance was measured using an enzyme linked immunoassay kit (ELISA kit).

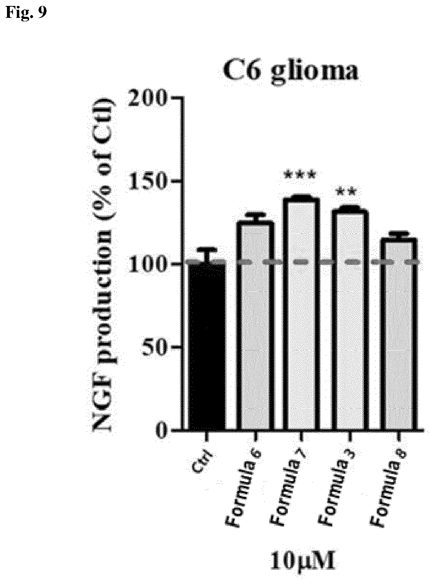

As shown in Fig. 9, it was found that the nerve growth factor produced in the cell medium was increased when treated with the compounds of the present invention, and in particular, it was found that the increased production of nerve growth factor was significantly excellent when treated with the compounds of Formulas 3 and 7.

### Evaluation of cytotoxicity

Rat astrocyte-derived C6 glioma cells were dispensed in DMEM culture solution at a concentration of 1 × 10⁵ cells/well in a 96 well plate and stabilized overnight, and then treated with the compounds of Formulas 3, 6, 7, and 8 at a concentration of 10 µM, and reacted for 24 hours. The culture solution was removed, and then 100 µl of MTT solution at a concentration of 0.5 mg/ml was added to each well and incubated for at least 1 hour in a 37 °C incubator, and then MTT was removed, and DMSO was dispensed in 200 µl, and absorbance of formazin produced in the well was measured at 540 nm using an ELISA reader.

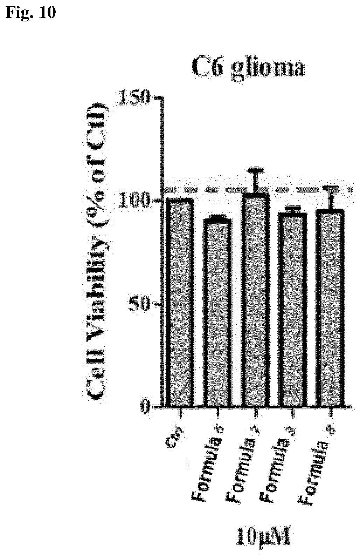

As shown in Fig. 10, when treated with all the compounds, there was no significant difference in cell viability from that of the control group, and thus, it was found that the compounds of the present invention did not exhibit cytotoxicity.

### [Experimental Example 2] Evaluation of ability to promote growth of nerve cells and cytotoxicity

In order to measure the growth degree of neurite and to confirm the effect on cell viability when N2a cells were treated with the compounds of the present invention, the following experiments were performed. Cerebrolysin, which has an effect of improving neurological dysfunction and is used as a therapeutic agent for diseases related to brain dysfunction, was used as a positive control group.

### Analysis of neurite growth

N2a cells (Neuro-2a cells) were coated with a solution of Poly-D-lysin (100 µg/ml) in a 24 well plate overnight, washed with sterile water, and then incubated for 1 hour. Nerve cells were dispensed in DMEM at a concentration of 15 ×10³ cells/well or 30 ×10³ cells/well and stabilized overnight, and then treated with the compounds of Formulas 3, 6, 7, and 8 at a concentration of 10 µM, and the growth of neurites was measured every 2 hours using the Incucyte zoom live cell analysis system.

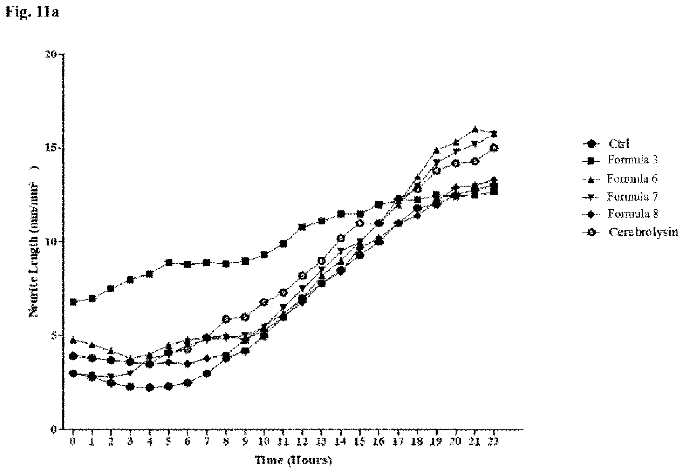

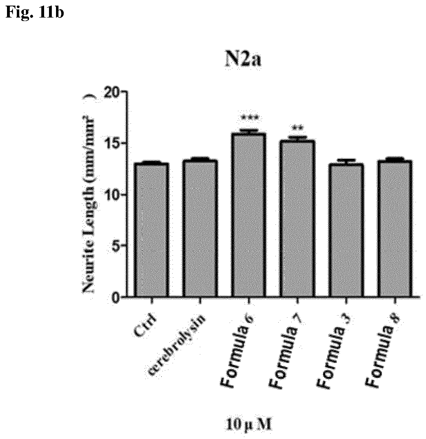

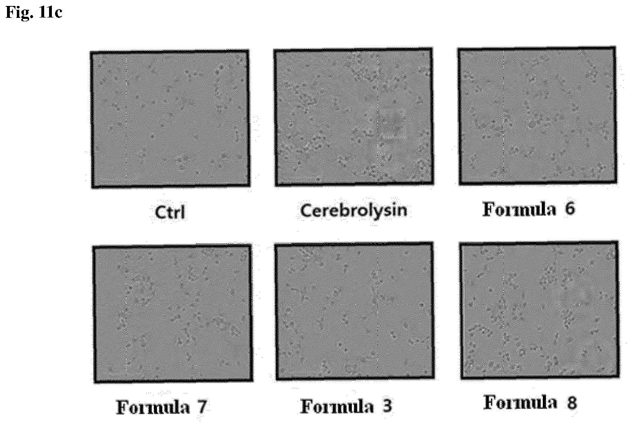

As shown in Figs. 11a to 11c, it was found that the growth of neurites was increased when treated with the compounds of the present invention. Therefore, it was found that the compounds of the present invention are effective for neurotrophic factors and promote the growth of nerve cells.

### Evaluation of cytotoxicity

N2a cells were dispensed in DMEM culture solution at a concentration of 1 × 10⁵ cells/well in a 96 well plate and stabilized overnight, and then treated with the compounds of Formulas 3, 6, 7, and 8 at a concentration of 10 µM, and reacted for 24 hours. The culture solution was removed, and then 100 µl of MTT solution at a concentration of 0.5 mg/ml was added to each well and incubated for at least 1 hour in a 37 °C incubator, and then MTT was removed, and DMSO was dispensed in 200 µl, and absorbance of formazin produced in each well was measured at 540 nm using an ELISA reader.

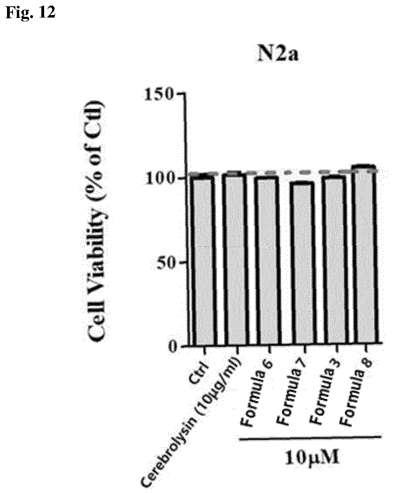

As shown in Fig. 12, when treated with all the compounds, there was no significant difference in cell viability from that of the control group, and thus, it was found that the compounds of the present invention did not exhibit cytotoxicity to nerve cells.

### [Experimental Example 3] Analysis of NO production and evaluation of cytotoxicity

In order to confirm the change in NO production and the effect on cell viability when BV2 cells were treated with the compounds of the present invention, the following experiments were performed. L-NMMA (NG-monomethyl-L-arginine), an NO synthesis inhibitor, was used as a positive control group.

### Analysis of NO production for evaluation of antineuritic activity

BV2 cells (microglia) were dispensed in DMEM culture solution at a concentration of 6 ×10⁴ cells/well in a 96 well plate, and then treated with the compounds of Formulas 6, 7, and 8 at a concentration of 10 µM, and incubated for 24 hours. After 24 hours, each sample was treated with a concentration of 10 µM, reacted for 1 hour, and then stimulated with 100 ng/ml of LPS for 24 hours. The production of nitrite, a soluble oxidation product of NO (nitric oxide), was measured in the culture medium through the Griess reaction.

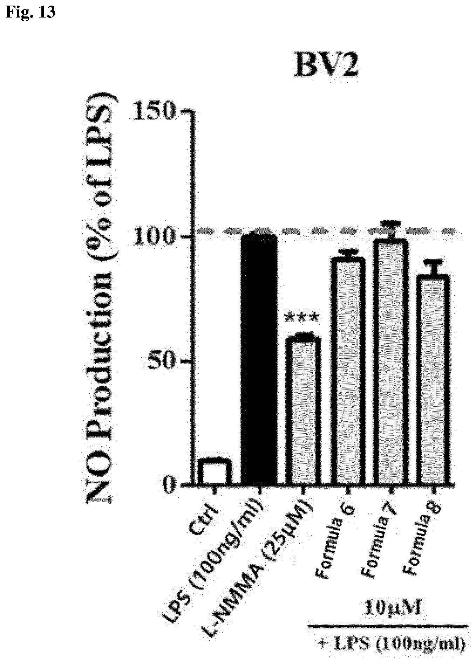

As shown in Fig. 13, it was found that when treated with the compounds of Formulas 6, 7, and 8, NO production was reduced compared to that of the untreated control group. Therefore, it was found that the compounds of the present invention have an antineuritic effect.

### Evaluation of cytotoxicity

BV2 cells were dispensed in DMEM culture solution at a concentration of 1 × 10⁵ cells/well in a 96 well plate and stabilized overnight, and then treated with the compounds of Formulas 3, 6, 7, and 8 at a concentration of 10 µM, and then reacted for 24 hours. The culture solution was removed, and then 100 µl of MTT solution at a concentration of 0.5 mg/ml was added to each well and incubated for at least 1 hour in a 37°C incubator, and then MTT was removed, and DMSO was dispensed in 200 µl, and absorbance of formazin produced in each well was measured at 540 nm using an ELISA reader.

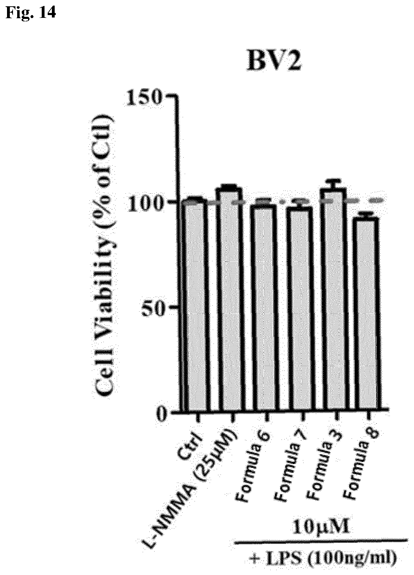

As shown in Fig. 14, when treated with all the compounds, there was no significant difference in cell viability from that of the control group, and thus, it was found that the compounds of the present invention did not exhibit cytotoxicity to microglia.

## Claims

1. A compound represented by Formula 9 below or a pharmaceutically acceptable salt thereof: 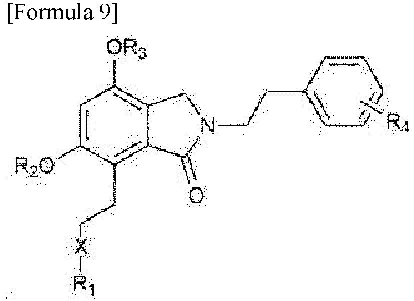 in the formula,
R₁ is hydrogen, alkyl, alkenyl, alkynyl, hydroxy, alkoxy, hydroxyalkyl, alkoxyalkyl, or -N(Ra)(Rb);
R₂, R₃, R₄, Ra and Rb are each independently hydrogen or alkyl; and
X is a single bond or a double bond.

2. The compound or pharmaceutically acceptable salt thereof according to claim 1, wherein
R₁ is hydrogen, alkyl, alkenyl, alkynyl, hydroxy, alkoxy, hydroxyalkyl, alkoxyalkyl, or -N(Ra)(Rb);
Ra and Rb are each independently hydrogen or alkyl;
R₂ and R₄ are hydrogen;
R₃ is alkyl; and
X is a single bond or a double bond.

3. The compound or pharmaceutically acceptable salt thereof according to claim 1, wherein
R₁ is C₁-C₆ alkyl, hydroxy, C₁-C₆ hydroxyalkyl, or -N(Ra)(Rb);
Ra and Rb are each independently C₁-C₆ alkyl;
R₂ and R₄ are hydrogen;
R₃ is C₁-C₆ alkyl; and
X is a single bond or a double bond.

4. A compound selected from the group consisting of compounds of the following formulas or a pharmaceutically acceptable salt thereof: 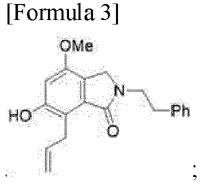 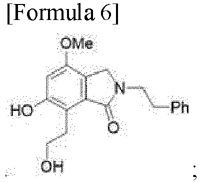 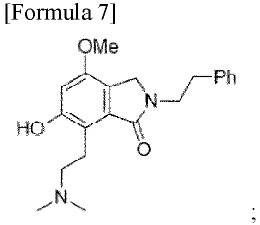 and

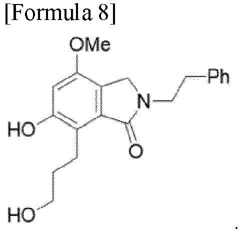

5. A pharmaceutical composition for preventing or treating neurological diseases, comprising a compound represented by Formula 9 below or a pharmaceutically acceptable salt thereof: 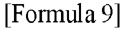 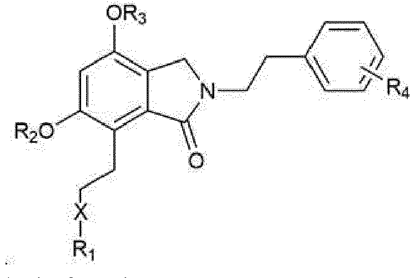 in the formula,
R₁ is hydrogen, alkyl, alkenyl, alkynyl, hydroxy, alkoxy, hydroxyalkyl, alkoxyalkyl, or -N(Ra)(Rb);
R₂, R₃, R₄, Ra and Rb are each independently hydrogen or alkyl; and
X is a single bond or a double bond.

6. The pharmaceutical composition for preventing or treating neurological diseases according to claim 5, wherein the neurological disease is selected from the group consisting of Alzheimer's disease, dementia, Parkinson's disease, epilepsy, neurological disorder, peripheral neuropathy, stroke and ischemic brain disease.

7. The pharmaceutical composition for preventing or treating neurological diseases according to claim 5, wherein the compound promotes the increase of nerve growth factor.

8. The pharmaceutical composition for preventing or treating neurological diseases according to claim 5, wherein the compound promotes the growth of nerve cells.

9. The pharmaceutical composition for preventing or treating neurological diseases according to claim 5, wherein the compound has antineuritic activity.

10. A food composition for preventing or improving neurological diseases, comprising a compound represented by Formula 9 below or a pharmaceutically acceptable salt thereof: 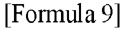 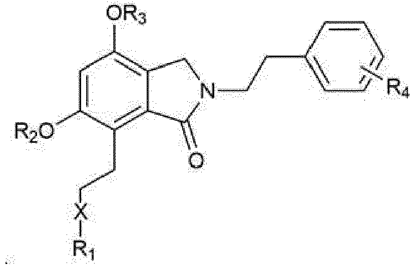 in the formula,
R₁ is hydrogen, alkyl, alkenyl, alkynyl, hydroxy, alkoxy, hydroxyalkyl, alkoxyalkyl, or -N(Ra)(Rb);
R₂, R₃, R₄, Ra and Rb are each independently hydrogen or alkyl; and
X is a single bond or a double bond.

11. The food composition for preventing or improving neurological diseases according to claim 10, wherein the neurological disease is selected from the group consisting of Alzheimer's disease, dementia, Parkinson's disease, epilepsy, neurological disorder, peripheral neuropathy, stroke and ischemic brain disease.

12. A feed composition for preventing or improving neurological diseases, comprising a compound represented by Formula 9 below or a pharmaceutically acceptable salt thereof: 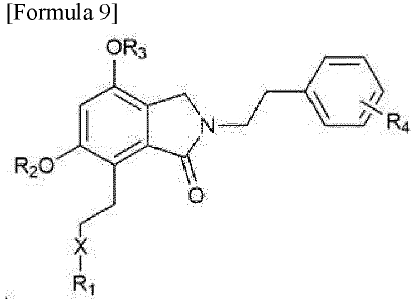 in the formula,
R₁ is hydrogen, alkyl, alkenyl, alkynyl, hydroxy, alkoxy, hydroxyalkyl, alkoxyalkyl, or -N(Ra)(Rb);
R₂, R₃, R₄, Ra and Rb are each independently hydrogen or alkyl; and
X is a single bond or a double bond.
